(19) Europäisches Patentamt
European Patent Office
Office européen des brevets

(11) Veröffentlichungsnummer : **0 095 641**
**B1**

(12) # EUROPÄISCHE PATENTSCHRIFT

(45) Veröffentlichungstag der Patentschrift : **29.07.87**

(51) Int. Cl.[4] : **C 07 D 487/04**, A 61 K 31/505

(21) Anmeldenummer : **83104794.9**

(22) Anmeldetag : **16.05.83**

(54) **Chinazolinonderivate, Verfahren zu ihrer Herstellung und ihre Verwendung in Arzneimitteln.**

(30) Priorität : **29.05.82 DE 3220438**

(43) Veröffentlichungstag der Anmeldung : **07.12.83 Patentblatt 83/49**

(45) Bekanntmachung des Hinweises auf die Patenterteilung : **29.07.87 Patentblatt 87/31**

(84) Benannte Vertragsstaaten : **AT BE CH DE FR GB IT LI NL SE**

(56) Entgegenhaltungen :
**DE-A- 2 008 998**
**DE-A- 2 234 174**
**DE-A- 2 257 376**
**DE-A- 2 402 454**
**US-A- 3 963 720**

(73) Patentinhaber : **Troponwerke GmbH & Co. KG**
**Berliner Strasse 156**
**D-5000 Köln 80 (DE)**

(72) Erfinder : **Opitz, Wolfgang, Dr.**
**Holzbachtalstrasse 60**
**D-5063 Overath (DE)**
Erfinder : **Jacobi, Haireddin, Dr.**
**Finkenweg 2**
**D-5653 Leichlingen (DE)**
Erfinder : **Pelster, Bernhard, Dr.**
**An den Weiden 7a**
**D-5201 St. Augustin 1 (DE)**

(74) Vertreter : **Jesse, Ralf-Rüdiger, Dr. et al**
**Bayer AG Konzernverwaltung RP Patentabteilung**
**D-5090 Leverkusen 1 Bayerwerk (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Chinazolinonderivate, Verfahren zu ihrer Herstellung sowie ihre Verwendung als Pharmazeutika.

In den DOS 2 234 174 und 2 257 376 sowie in den US-Patenten 3 887 559, 3 905 976, 3 969 506, 3 982 000 und 3 984 556 sind für strukturell ähnliche Verbindungen bronchodilatorische, hypotensive und Wirkungen auf das zentrale Nervensystem beschrieben.

In der DE-A-24 02 454 werden Imidazo-chinazolin-one genannt, die als Bronchodilatoren verwendet werden können. Die in der US 3 963 720 beschriebenen Imidazo-chinazolin-one sind ebenfalls Bronchodilatoren und haben eine Wirkung als Beruhigungsmittel.

Gegenstand der DE-A-2 257 375 und der DE-A-2 234 174 sind Verfahren zur Herstellung von Imidazo-chinazolin-onen aus Isantinsäureanhydrid oder Chinazolin-5 (1 OH)onen. Die in der DE-A-2 008 998 Imidazo-[2,1-b]-chinazoline können als Sedativa, Tranquillizer oder Anxiolytika verwendet werden.

Es wurden neue Chinazolinonderivate der allgemeinen Formel

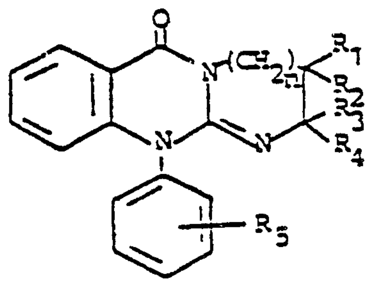

a) in welcher $R_1$, $R_2$, $R_3$ und $R_4$ jeweils für ein Wasserstoffatom und n für 0 oder 1 oder

b) $R_1$ und $R_3$ für ein Wasserstoffatom und $R_2$ und $R_4$ gemeinsam für eine Bindung und n für 0 oder

c) $R_1$ und $R_3$ gemeinsam für eine Bindung und $R_2$ und $R_4$ gemeinsam für die Gruppierung

und n für 0 stehen

und in welcher

$R_5$ für Alkylreste mit 1 bis 4 C-Atomen, die vollständig oder teilweise durch Halogenatome substituiert sind, für Alkylthiogruppen mit 1 bis 3 C-Atomen, für Alkylsulfinyl mit 1 bis 3 C-Atomen, für Alkylsulfonyl mit 1 bis 3 C-Atomen, für Nitro- oder für freie sowie durch 1 oder 2 Alkylreste mit 1 bis 3 C-Atomen oder durch einen 1 bis 3 C-Atome enthaltenden Acylrest substituierte Aminogruppen

steht, sowie ihre physiologisch unbedenklichen Salze mit Säuren.

Überraschenderweise wurde nun gefunden, daß die neuen Chinazolinonderivate interessante entzündungshemmende Wirkungen besitzen. Einzelne Verbindungen sind — ähnlich wie für die in den genannten Anmeldungen enthaltenden Verbindungen ohne Angabe von Meßergebnissen behauptet — zentral aktiv ; sie zeichnen sich durch starke analgetische und sedierende Wirkungen aus.

Von besonderem Interesse sind Verbindungen der allgemeinen Formel I,

in der $R_1$, $R_2$, $R_3$ und $R_4$ jeweils für ein Wasserstoffatom und n für 0 oder 1, oder

$R_1$ und $R_3$ für ein Wasserstoffatom und $R_2$ und $R_4$ gemeinsam für eine Bindung und n für 0 oder

$R_1$ und $R_3$ gemeinsam für eine Bindung und $R_2$ und $R_4$ gemeinsam für die Gruppierung

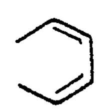

und n für 0 stehen und in welcher

$R_5$ für eine Trifluormethyl-, eine Methylthio-, eine Methylsulfonyl-, Nitro- oder Aminogruppe steht.

Zu den erfindungsgemäßen Verbindungen der allgemeinen Formel I, in der $R_1$, $R_2$, $R_3$ und $R_4$ jeweils für Wasserstoff und n für 0 oder 1

oder $R_1$ und $R_3$ gemeinsam für eine Bindung und $R_2$ und $R_4$ gemeinsam für die Gruppierung

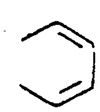

und n für 0 sowie $R_5$ für halogenierte Alkyl-, Alkylthio- oder Nitrogruppen steht, gelangt man, indem man die entsprechenden N-Arylanthranilsäuren II mit geeigneten Aktivierungsreagenzien in ein aktiviertes Carbonsäurederivat III überführt (eine Übersicht findet sich z. B. in Houben-Weyl « Methoden der Organischen Chemie », Band 15/2, Georg Thieme Verlag Stuttgart, 1974).

Erfindungsgemäß bevorzugt wird die Umsetzung von II mit $PCl_5$ (III : x = Cl ;

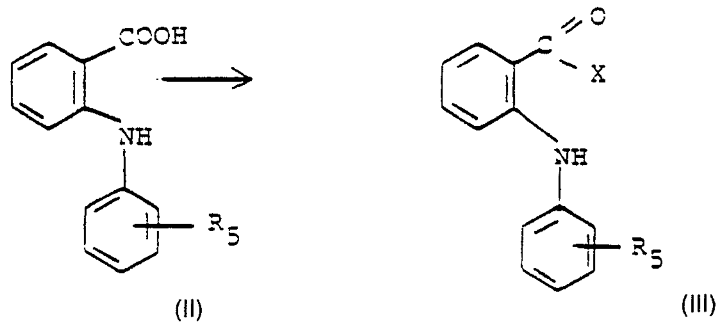

(II) (III)

vgl. J. Amer. Chem. Soc. 68, 2112 (1946)) oder mit Dicyclohexylcarbodiimid und Pentachlorphenol (III : $X = OC_6Cl_5$ ; vgl. Helv. Chim. Acta 46, 1609 (1963)).

Die so erhaltenen Anthranilsäurederivate III können entweder als reine isolierte Verbindungen oder ohne vorherige Isolierung in geeigneten aprotischen Lösungsmitteln, wie z. B. Dimethylformamid, Dioxan oder THF sowie Gemischen dieser Lösungsmittel, bevorzugt ist Dioxan, mit literaturbekannten S-alkylierten, bevorzugt methylierten, cyclischen Isothioharnstoffderivaten IV

(III) +

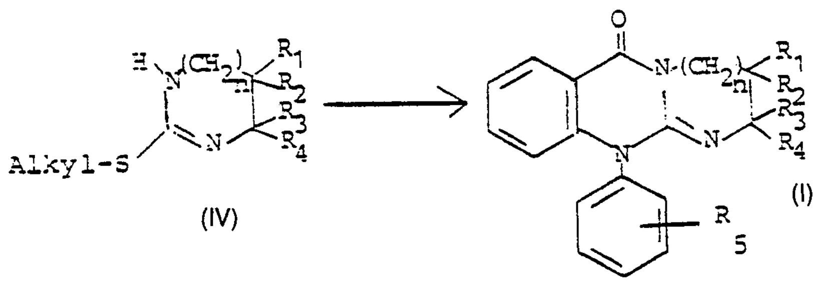

(IV) (I)

in denen $R_1$, $R_2$, $R_3$ und $R_4$ jeweils für Wasserstoff und n für 0 oder 1 oder $R_1$ und $R_3$ gemeinsam für eine Bindung und $R_2$ und $R_4$ gemeinsam für die Gruppierung

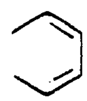

und n für 0 stehen, zu den entsprechenden erfindungsgemäßen Verbindungen der allgemeinen Formel I umgesetzt werden.

Die Reaktion wird bei erhöhten Temperaturen vorgenommen, vorzugsweise bei Temperaturen von etwa 40 °C unterhalb der Siedetemperatur des Lösungsmittels bis einschließlich zur Siedetemperatur der verwendeten Lösungen. Ganz besonders bevorzugt wird bei der Siedetemperatur der verwendeten Lösung gearbeitet. Es ist zwar möglich, auch bei noch niedrigeren Temperaturen zu arbeiten, jedoch wird dann für die Umsetzung eine längere Zeit benötigt.

Aus den so erhaltenen Verbindungen der allgemeinen Formel I, in denen $R_5$ für Alkylthio steht, lassen sich mit geeigneten Oxidationsmitteln, bevorzugt Wasserstoffperoxid, weitere erfindungsgemäße Verbindungen der allgemeinen Formel I, in denen $R_5$ für Alkylsulfinyl oder Alkylsulfonyl steht, herstellen.

Die Umsetzung der erfindungsgemäßen Verbindungen der allgemeinen Formel I, in denen $R_1$, $R_2$, $R_3$ und $R_4$ jeweils für Waserstoffatom und n für 0 steht, mit Oxidationsmitteln, wie z. B. aktivem Braunstein (vgl. J. Org. Chem. 33, 3758 (1968)) ergibt erfindungsgemäße Verbindungen der allgemeinen Formel I, in denen $R_1$ und $R_3$ für ein Wasserstoffatom und $R_2$ und $R_4$ gemeinsam für eine Bindung und n für 0 steht.

Die Reduktion, z. B. durch katalytische Hydrierung, der erfindungsgemäßen Verbindungen der allgemeinen Formel I, in denen $R_5$ für Nitro steht, ergibt erfindungsgemäße Verbindungen der allgemeinen Formel I, in denen $R_5$ für Amino steht.

Überraschenderweise zeigen die erfindungsgemäßen Verbindungen der allgemeinen Formel I zum Teil sehr starke entzündungshemmende, analgetische und sedierende Wirkungen bei vergleichsweise geringer Toxizität und stellen somit eine Bereicherung der Pharmazie dar.

Als Beispiele für die erfindungsgemäßen Verbindungen der allgemeinen Formel I seien im einzelnen genannt :

10-(3-Methylthiophenyl)-2,3-dihydroimidazo [2,2-b]-chinazolin-5 (10H)on

10-(4-Methylthiophenyl)-2,3-dihydroimidazo [2,1-b]-chinazolin-5 (10H)on

10-(3-Nitrophenyl)-2,3-dihydroimidazo [2,1-b]-chinazolin-5 (10H)on

10-(3-Trifluormethyl)-2,3-dihydroimidazo [2,1-b]-chinazolin-5 (10H)on

10-(3-Aminophenyl)-2,3-dihydroimidazo-[2,1-b]-chinazolin-5 (10H)on

10-(4-Methylsulfinylphenyl)-2,3-dihydroimidazo-[2,1-b]-chinazolin-5 (10H)on

10-(3-Trifluormethylphenyl)-imidazo [2,1-b]-chinazolin-5 (10H)on

5-(3-Nitrophenyl)-benzimidazo [2,1-b]-chinazolin-12 (5H)on

11-(3-Nitrophenyl)-2,3,4,6-tetrahydropyrimido [2,1-b]-chinazolin-6on.

Der Nachweis der antiphlogistischen Wirkung erfolgte nach der Methode von Winter et al., Proc. Soc. Exp. Biol. Med. 111, 544 (1962) mit der Abwandlung, daß zur Ödemprovokation reines λ-Carrageenan als 0,25 %ige Suspension in physiologischer Kochsalzlösung verwendet wurde. Beispielhaft wurden folgende $DE_{50}$-Werte ermittelt :

| Verbindung aus Beispiel | $DE_{50}$ Carrageenanödem p.o. |
|---|---|
| 4 | 1,3 mg/kg |
| 5 | 25,2 " |
| 9 | 3,1 " |

Zum Nachweis der sedativen Wirkung wurde die Beeinflussung der Orientierungsmotilität bei der Maus geprüft ; für die Verbindung aus Beispiel 4 wurde eine $DE_{50}$ von 0,5 mg/kg p.o. bestimmt. Die $DE_{50}$ von Diazepam im gleichen Test ist 2,8 mg/kg p.o., von Chlorpromazin 3,05 mg/kg p.o.

Die analgetische Wirkung für die Verbindung aus Beispiel 4 wurde im Randall-Selitto-Test ($DE_{50}$ 3,4 mg/kg p.o., Ratte) sowie im Benzochinon-Test ($DE_{50}$ 3,1 mg/kg p.o. ; Maus) nachgewiesen.

Zur vorliegenden Erfindung gehören pharmazeutische Zubereitungen, die neben nichttoxischen, inerten, pharmazeutisch geeigneten Trägerstoffen einen oder mehrere erfindungsgemäße Wirkstoffe enthalten, oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagsdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie Füll- und Streckmittel (z. B. Stärken, Milchzucker, Rohrzucker, Glukose, Mannit und Kieselsäure), Bindemittel (z. B. Carboxymethylcellulose, Alginate, Gelatine, Polyvinylpyrrolidon), Feuchthaltemittel (z. B. Glycerin), Sprengmittel (z. B. Agar-Agar, Calciumlcarbonat und Natriumbicarbonat), Lösungsverzögerer (z. B. Paraffin) und Resorptionsbeschleuniger (z. B. quaternäre Ammoniumverbindungen), Adsorptionsmittel (z. B. Kaolin und Bentonit) und Gleitmittel (z. B. Talkum, Calcium- und Magnesiumstearat und feste Polyethylenglykole) oder Gemische der aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert, abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen zur Erzielung eines Retardeffektes.

Suppositorien können neben dem oder den Wirkstoffen die üblichen wasserlöslichen oder wasserunlöslichen Trägerstoffe enthalten, z. B. Polyethylenglykole, Fette z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoff die üblichen Trägerstoffe enthalten (z. B. tierische und pflanzliche Fette, Wachse, Paraffine, Stärken, Tragant) oder Gemische dieser Stoffe.

Puder und Sprays können neben dem oder den Wirkstoffen die üblichen Trägerstoffe enthalten (z. B. Milchzucker, Talkum, Kieselsäure, Aluminiumhydroxid) oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, Öle, insbesondere Baumwollsaatöl, Erdnußöl, Maiskeimöl, Olivenöl und Sesamöl, Glycerin, Polyethylenglykole oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel (z. B. Wasser, Ethylalkohol, Propylenlykol), Suspendiermittel (z. B. ethoxylierte Isostearylalkohole, Polyoxyethylensorbit- und Sorbitanester, mikrokristalline Cellulose) oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung entzündlicher Prozesse im menschlichen und tierischen Organismus.

Die Wirkstoffe oder die pharmazeutischen Zubereitungen können cutan, oral, parenteral, intraperitoneal und/oder rectal, vorzugsweise oral oder cutan, appliziert werden.

Im allgemeinen ist es in der Humanmedizin als vorteilhaft anzusehen, den oder die erfindungsgemäßen Wirkstoffe in Gesamtmengen von etwa 0,1 bis 200, vorzugsweise 0,1 bis 70 mg je 24 Stunden, gegebenenfalls in Form mehrerer Einzelgaben zur Erzielung der gewünschten Ergebnisse, zu verabreichen.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen fällen die oben angeführte Wirkstoffmenge überschritten werden muß.

Die Festlegung der jeweils erforderlichen optimalen Dosierung und Applikationsart der Wirkstoffe kann durch jeden Fachmann aufgrund seines Fachwissens leicht erfolgen.

Die vorliegende Erfindung soll durch die nachfolgenden Beispiele noch weiter erläutert werden.

## Beispiele

1. 2-(3-Trifluormethylphenylamino)benzoylchlorid

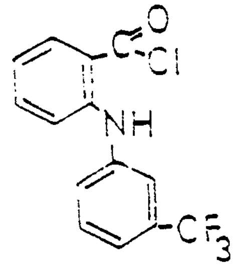

Eine Suspension von 100 g (0,356 Mol) 2-(3-Trifluormethylphenylamino)benzoesäure in 175 ml Toluol wird unter Rühren und Stickstoffspülung bei 5-10 °C portionsweise mit 78,5 g (0,376 Mol) $PCl_5$ versetzt und 30 Minuten unter Kühlung nachgerührt. Die Reaktionslösung wird unter Stickstoff und Eiskühlung fünfmal mit je 50 ml Wasser unter kräftigem Durchrühren gewaschen, über $Na_2SO_4$ getrocknet, bei 40° im Vakuum eingedampft und der ölige Eindampfrückstand mit Petrolether versetzt. In der Kälte kristallisiert das Chlorid aus. Ausbeute : 90,4 g (85 % der Theorie), Schmelzpunkt 63-65 °C.

2. In ähnlicher Weise wird aus 2-(3-Nitrophenylamino)-benzoesäure erhalten :

2-(3-Nitrophenylamino) benzoylchlorid

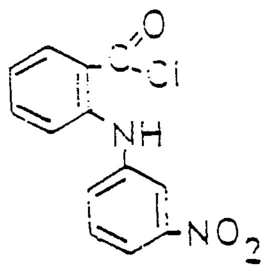

Ausbeute : 77 % der Theorie, Schmelzpunkt : 141-145 °C.

3. 10-(4-Methylthiophenyl)-2,3-dihydroimidazo-[2,1-b]-chinazolin-5 (10H) on

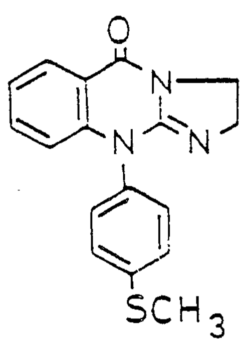

7,8 g (30 m Mol) 2-(4-Methylthiophenylamino)-benzoesäure werden in 150 ml n-Hexan suspendiert, mit 7,1 g (34 m Mol) $PCl_5$ versetzt und bei Raumtemperatur 2 Stunden gerührt und anschließend über Nacht aufbewahrt. Man filtriert, löst den Niederschlag in 120 ml Dioxan, versetzt mit 3,8 g (33 m Mol) 2-Methylthioimidazolin, kocht 90 Minuten unter Rückfluß und verdampft das Lösungsmittel im Vakuum. Der Rückstand wird mit Wasser versetzt, mit 2 n HCl angesäuert und filtriert. Die Lösung wird mit 2 n NaOH auf pH 9-10 gestellt und mit Dichlormethan extrahiert. Nach Trocknen und Eindampfen der organischen Phase wird aus Dichlormethan/Petrolether kristallisiert. Ausbeute : 4,5 g (48,5 % der Theorie), Schmelzpunkt 226-227 °C ; N ber. 13,58 % ; N gef. 13,45 %.

4. 10-(3-Nitrophenyl)-2,3-dihydroimidazo-[2,1b]-chinazolin-5(10H) on

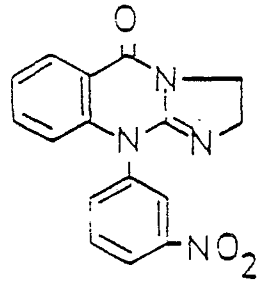

Eine Suspension von 11 g (40 m Mol) 2-(3-Nitrophenylamino) benzoylchlorid und 5,1 g (44 m Mol) 2-Methylthioimidazolin in 160 ml Dioxan wird 1 Stunde unter Rückfluß gekocht, das Lösungsmittel im Vakuum verdampft und der Rückstand zwischen Dichlormethan und ges. $KHCO_3$-Lösung verteilt. Die organische Phase wird über $Na_2SO_4$ getrocknet, eingedampft und der Rückstand aus Dichlormethan durch Zusatz von Diisopropylether kristallisiert. Ausbeute : 5,8 g (47 % der Theorie), Schmelzpunkt 275-277 °C ; N ber. 18,17 % ; N gef. 18,47 %.

5. 10-(3-Trifluormethylphenyl)-2,3-dihydroimidazo-[2,1-b]-chinazolin-5(10H) on

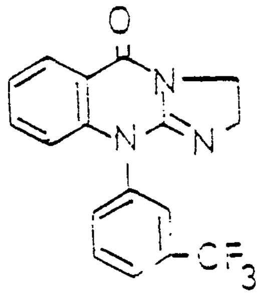

Eine Suspension aus 6 g (20 m Mol) 2-(3-Trifluormethylphenylamino)-benzoylchlorid und 4,9 g (20 m Mol) 2-Methylthioimidazolin-Hydroiodid in 50 ml Dioxan wird bei Raumtemperatur mit 2,8 ml (20 mMol) Triethylamin in 10 ml Dioxan versetzt und das Gemisch 2 Stunden unter Rückfluß gekocht. Das Lösungsmittel wird abgezogen, der Rückstand zwischen Dichlormethan und wäßriger NaOH (pH 9-10) verteilt, die organische Phase über $Na_2SO_4$ getrocknet, eingedampft und der verbleibende Rückstand aus Dichlormethan/Diisopropylether kristallisiert. Ausbeute : 3,65 g (55 % der Theorie), Schmelzpunkt 251-252 °C ; N ber. 12,68 % ; N gef. 12,72 %.

6. Analog wurde aus 2-(3-Nitrophenylamino) benzoylchlorid und 2-Methyl-thio-$\Delta^2$-tetrahydropyrimidin-Hydroiodid (J. Amer. Chem. Soc. 78, 1618 (1956) hergestellt :

11-(3-Nitrophenyl)-2,3,4,6-tetrahydropyrimido [2,1-b]-chinazolin-6 on

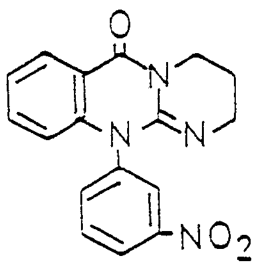

Ausbeute : 43 % der Theorie, Schmelzpunkt 249-250 °C ; N ber. 17,36 % ; N gef. 17,51 % sowie

7. aus 2-(3-Nitrophenylamino) benzoylchlorid und 2-Methylthiobenzimidazol :

5-(3-Nitrophenyl)-benzimidazo-[2,1-b]-chinazolin-12 (5H) on

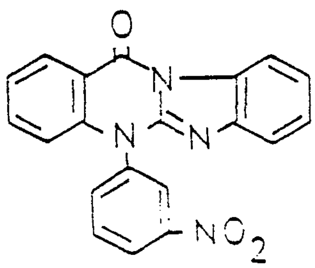

Ausbeute : 62 % der Theorie, Schmelzpunkt 294 °C ; N Ber. 15,72 % ; N gef. 15,73 %.

8. 10-(3-Methylthiophenyl)-2,3-dihydroimidazo-[2,1-b]-chinazolin-5 (10H) on

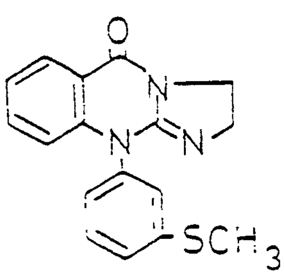

Eine Lösung von 16,3 g (63 m Mol) 2-(3-Methylthiophenylamino) benzoesäure und 16,8 g (63 m Mol)

Pentachlorphenol in 80 ml DMF wird mit einer zweiten Lösung von 13 g (63 m Mol) Dicyclohexylcarbodiimid in 20 ml DMF vereinigt und das Reaktionsgemisch 2 Stunden bei Raumtemperatur aufbewahrt. Man filtriert ausgefallenen Dicyclohexylharnstoff ab und versetzt das Filtrat mit einem Gemisch aus 15,4 g (63 m Mol) 2-Methylthioimidazolin-Hydroiodid und 8,7 ml (63 m Mol) Triethylamin in 40 ml DMF. Nach Aufbewahren über Nacht wird das Reaktionsgemisch 4 Stunden auf 50 °C erwärmt, mit Eiswasser versetzt und der entstehende Niederschlag abfiltriert. Zur Reinigung wird der Festkörper zwischen Dichlormethan und 2n NaOH verteilt, die organische Phase über $Na_2SO_4$ getrocknet, eingedampft und der Rückstand aus Dichlormethan/Diisopropylather kristallisiert. Ausbeute : 7,8 g (40 % der Theorie), Schmelzpunkt 222-223 °C ; N ber. 13,58 %, N gef. 13,48 %.

9. 10-(3-Aminophenyl)-2,3-dihydroimidazo-[2,1-b]-chinazolin-5 (10H) on

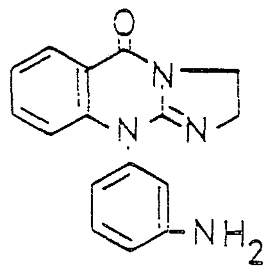

Eine Suspension von 9,9 g (32 m Mol) 10-(3-Nitrophenyl)-2,3-dihydroimidazo-[2,1-b] chinazolin-5 (10H) on und 2 g Pd-Kohle (10 %) in 500 ml Methanol wird mit 20 ml (0,413 Mol) 100 %igem Hydrazinhydrat versetzt und 6 Stunden unter Rückfluß gekocht.

Nach Zugabe von jeweils der gleichen Menge Katalysator und Hydrazinhydrat wird erneut 6 Stunden gekocht, heiß filtriert, das Filtrat eingedampft und der Rückstand mit Wasser ausgerührt. Ausbeute : 3 g (34 % der Theorie), Schmelzpunkt 309-311 °C ; N ber. 20,13 % ; N gef. 20,13 %.

10. 10-(4-Methylsulfinylphenyl)-2,3-dihydroimidazo-[2,1-b]-chinazolin-5 (10H) on -Monohydrat

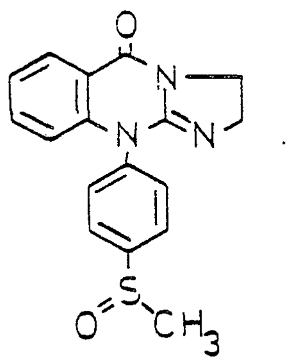

Eine Lösung von 2,04 g (6,6 m Mol) 10-(4-Methylthiophenyl)-2,3-dihydroimidazo-[2,1-b]-chinazolin-5 (10H) on in 20 ml Eisessig wird nach Zugabe von 0,77 ml 30 %igem Wasserstoffperoxid zunächst 22 Std. und nach Zugabe von weiteren 0,2 ml 30 %igem Wasserstoffperoxid weitere 7 Stunden bei Raumtemperatur aufbewahrt. Peroxidüberschuß wird durch Zusatz einer Spateilspitze Pt-Mohr zerstört, der Ansatz filtriert, das Filtrat eingedampft. Der Rückstand wird in Wasser aufgenommen und die Lösung mit ges. $KHCO_3$-Lösung versetzt. Der entstandene Niederschlag wird abfiltriert und an Aluminiumoxid 90 (Merck) zuerst mit Essigester und anschließend mit Dichlormethan/Methanol (95 : 5) chromatographiert. Die produkthaltigen Fraktionen werden eingedampft und der erhaltene Rückstand aus Dichlormethan/Diisopropylether kristallisiert. Ausbeute : 1,6 g (71 % der Theorie), Schmelzpunkt 257-260 °C, N ber. 12,24 % ; N gef. 12,01 %.

11. 10-(3-Trifluormethyl)-imidazo [2,1-b]-chinazolin-5 (10H) on

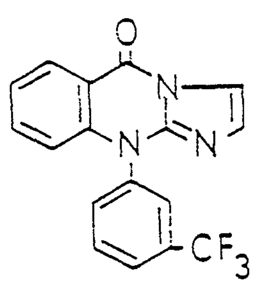

Eine Lösung von 1,65 g (5 m Mol) 10-(3-Trifluormethyl)-2,3-dihydroimidazo-[2,1-b]-chinazolin-5 (10H) on in 30 ml abs. Dichlormethan wird mit 7 g aktivem Braunstein versetzt, über Nacht bei Raumtemperatur aufbewahrt und 90 Minuten unter Rückfluß gekocht. Es wird filtriert, das Filtrat eingedampft und der Rückstand mit Essigester an Kieselgel chromatographiert. Ausbeute : 0,5 g (30 % der Theorie) und 0,95 g Mischfraktionen, Schmelzpunkt 206-208 °C ; N ber. 12,78 % ; N gef. 12,69 %.

**Patentansprüche**

1. Chinazolinonderivate der allgemeinen Formel

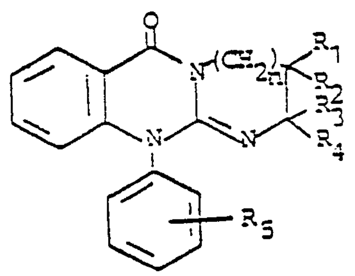

a) in welcher $R_1$, $R_2$, $R_3$ und $R_4$ jeweils für ein Wasserstoffatom und n für 0 oder 1 oder
b) $R_1$ und $R_3$ für ein Wasserstoffatom und $R_2$ und $R_4$ gemeinsam für eine Bindung und n für 0 oder
c) $R_1$ und $R_3$ gemeinsam für eine Bindung und $R_2$ und $R_4$ gemeinsam für die Gruppierung

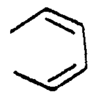

und n für 0 stehen
und in welcher
$R_5$ für Alkylreste mit 1 bis 4 C-Atomen, die vollständig oder teilweise durch Halogenatome substituiert sind, für Alkylthiogruppen mit 1 bis 3 C-Atomen, für Alkylsulfinyl mit 1 bis 3 C-Atomen, für Alkylsulfonyl mit 1 bis 3 C-Atomen, für Nitro- oder für freie sowie durch 1 oder 2 Alkylreste mit 1 bis 3 C-Atomen oder durch einen 1 bis 3 C-Atome enthaltenden Acylrest substituierte Aminogruppen
steht, sowie ihre physiologisch unbedenklichen Salze mit Säuren.

2. Chinazolinonderivate nach Anspruch 1, wobei
$R_1$, $R_2$, $R_3$ und $R_4$ jeweils für Wasserstoff und n für 0 oder 1
oder
$R_1$ und $R_3$ für Wasserstoff und $R_2$ und $R_4$ gemeinsam für eine Bindung und n für 0
oder
$R_1$ und $R_3$ gemeinsam für eine Bindung und
$R_2$ und $R_4$ gemeinsam für die Gruppierung

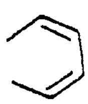

und n für 0 stehen und
$R_5$ für eine Trifluormethyl-, Methylthio-, Methylsulfonyl-, Nitro- oder Aminogruppe steht.

3. Verfahren zur Herstellung von Chinazolinonderivaten nach Anspruch 1, dadurch gekennzeichnet, daß man N-Arylanthranilsäuren mit Aktivierungsreagenzien zunächst in aktivierte Derivate überführt und diese anschließend in aprotischen Lösungsmitteln mit S-alkylierten cyclischen Isothioharnstoffderivaten bei erhöhten Temperaturen umsetzt.

4. Verfahren nach Anspruch 3, dadurch gekennzeichnet, daß als Aktivierungsreagenzien Phosphorpentachlorid oder Dicyclohexylcarbodiimid und Pentachlorphenol, als aprotische Lösungsmittel Dimethylformamid, Dioxan oder Tetrahydrofuran oder ihre Gemische und als S-alkylierte cyclische Isothioharnstoffderivate solche, in denen
$R_1$, $R_2$, $R_3$ und $R_4$ jeweils für Wasserstoff und n für 0 oder 1
oder
$R_1$ und $R_3$ gemeinsam für eine Bindung und $R_2$ und $R_4$ gemeinsam für die Gruppierung

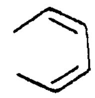

und n für 0 stehen eingesetzt werden.

5. Verfahren nach Anspruch 3 und 4, dadurch gekennzeichnet, daß die Umsetzung bei der Siedetemperatur der Lösung vorgenommen wird.

6. Chinazolinonderivate nach Anspruch 1 zur Bekämpfung von Krankheiten, insbesondere Bekämpfung von Entzündungen, Schmerzen und Überreaktionen des zentralen Nervensystems.

7. Arzneimittel enthaltend mindestens ein Chinazolinonderivat nach Anspruch 1.

8. Verwendung von Chinazolinonderivaten nach Anspruch 1 zur Herstellung von Arzneimitteln.

9. Verwendung nach Anspruch 8 zur Herstellung von Entzündungshemmern, Analgetika und/oder Sedativa.

**Claims**

1. Quinazolinone derivatives of the general formula

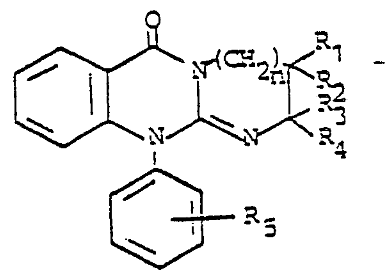

a) in which $R_1$, $R_2$, $R_3$ and $R_4$ each represent a hydrogen atom and n represents 0 or 1 or

b) $R_1$ and $R_3$ represent a hydrogen atom and $R_2$ and $R_4$ together represent a bond and n represents 0 or

c) $R_1$ and $R_3$ together represent a bond and $R_2$ and $R_4$ together represent the grouping

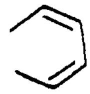

and n represents 0
and in which

$R_5$ represents alkyl radicals with 1 to 4 C atoms, which are completely or partially substituted by halogen atoms, alkylthio groups with 1 to 3 C atoms, alkylsulphinyl with 1 to 3 C atoms, alkylsulphonyl with 1 to 3 C atoms, nitro or free amino groups or amino groups which are substituted by 1 or 2 alkyl radicals with 1 to 3 C atoms or by an acyl radical containing 1 to 3 C atoms,
and their physiologically acceptable salts with acids.

2. Quinazolinone derivatives according to Claim 1, wherein

$R_1$, $R_2$, $R_3$ and $R_4$ each represent hydrogen and n represents 0 or 1

or

$R_1$ and $R_3$ represent hydrogen and $R_2$ and $R_4$ together represent a bond and n represents 0

or

$R_1$ and $R_3$ together represent a bond and
$R_2$ and $R_4$ together represent the grouping

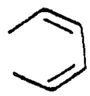

and n represents 0 and

$R_5$ represents a trifluoromethyl, methylthio, methylsulphonyl, nitro or amino group.

3. Process for the preparation of quinazolinone derivatives according to Claim 1, characterised in that N-arylanthranilic acids are first converted into activated derivatives with activating reagents and these derivatives are then reacted with S-alkylated cyclic isothiourea derivatives in aprotic solvents at elevated temperatures.

4. Process according to Claim 3, characterised in that phosphorus pentachloride or dicyclohexylcarbodiimide and pentachlorophenol are used as activating reagents, dimethyl formamide, dioxan or

tetrahydrofuran or mixtures thereof are used as aprotic solvents and the S-alkylated cyclic isothiourea derivatives used are those in which

$R_1$, $R_2$, $R_3$ and $R_4$ each represent hydrogen and n represents 0 or 1,

or

$R_1$ and $R_3$ together represent a bond and $R_2$ and $R_4$ together represent the grouping

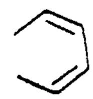

and n represents 0.

5. Process according to Claim 3 and 4, characterised in that the reaction is carried out at the boiling temperature of the solution.

6. Quinazolinone derivatives according to Claim 1 for combating diseases, in particular for combating inflammations, pain and hyperintensive reactions of the central nervous system.

7. Medicaments containing at least one quinazolinone derivative according to Claim 1.

8. Use of quinazolinone derivatives according to Claim 1 for the preparation of medicaments.

9. Use according to Claim 8 for the preparation of inflammation inhibitors, analgesics and/or sedatives.

**Revendications**

1. Dérivés de la quinazolinone de formule générale

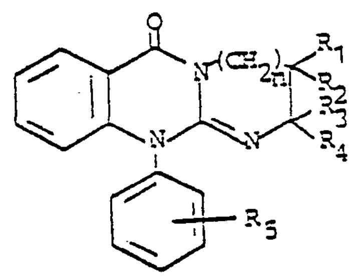

a) dans laquelle $R_1$, $R_2$, $R_3$ et $R_4$ représentent chacun un atome d'hydrogène et n est égal à 0 ou 1, ou bien

b) $R_1$ et $R_3$ représentent chacun un atome d'hydrogène, et $R_2$ et $R_4$ représentent ensemble une liaison, et n est égal à 0, ou bien

c) $R_1$ et $R_3$ représentent ensemble une liaison et $R_2$ et $R_4$ représentent ensemble le groupement

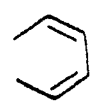

et n est égal à 0,

et dans laquelle

$R_5$ représente des groupes alkyle en $C_1$-$C_4$ substitués en totalité ou en partie par des atomes d'halogènes, des groupes alkylthio en $C_1$-$C_3$, un groupe alkylsulfinyle en $C_1$-$C_3$, un groupe alkylsulfonyle en $C_1$-$C_3$, un groupe nitro, ou bien des groupes amino libres ou substitués par 1 ou 2 groupes alkyle en $C_1$-$C_3$ ou par un groupe acyle en $C_1$-$C_3$,

et leurs sels d'acides acceptables pour l'usage pharmaceutique.

2. Dérivés de la quinazolinone selon la revendication 1, dans lesquels

$R_1$, $R_2$, $R_3$ et $R_4$ représentent chacun l'hydrogène et n est égal à 0 ou 1,

ou bien

$R_1$ et $R_3$ représentent l'hydrogène et $R_2$ et $R_4$, ensemble, une liaison, et n est égal à 0,

ou bien

$R_1$ et $R_3$ représentent ensemble une liaison, et

$R_2$ et $R_4$ représentent ensemble le groupement

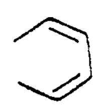

et n est égal à 0, et

$R_5$ représente un groupe trifluorométhyle, méthylthio, méthylsulfonyle, nitro ou amino.

3. Procédé de préparation des dérivés de la quinazolinone selon le revendication 1, caractérisé en ce qu'on convertit d'abord des acides N-arylanthraniliques, à l'aide de réactifs activants, en dérivés activés qu'on fait ensuite réagir dans des solvants aprotoniques, à chaud, avec des dérivés cycliques de l'isothiourée alkylés sur le soufre.

4. Procédé selon la revendication 3, caractérisé en ce que l'on utilise en tant que réactifs activants le pentachorure de phosphore ou le dicyclohexylcarbodiimide et le pentachlorophénol, en tant que solvants aprotoniques le diméthylformamide, le dioxanne ou le tétrahydrofuranne ou leurs mélanges, et en tant que dérivés cycliques de l'isothiourée alkylés sur le soufre des dérivés de ce type dans lesquels $R_1$, $R_2$, $R_3$ et $R_4$ représentent chacun l'hydrogène et n est égal à 0 ou 1,

ou bien

$R_1$ et $R_3$ représentent ensemble une liaison et $R_2$ et $R_4$ représentent ensemble le groupement

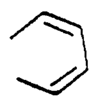

et n est égal à 0.

5. Procédé selon les revendications 3 et 4, caractérisé en ce que la réaction est effectuée à la température d'ébullition de la solution.

6. Dérivés de la quinazolinone selon la revendication 1, pour le traitement de maladies, en particulier pour le traitement des inflammations, des douleurs et des réactions excessives du système nerveux central.

7. Médicaments contenant au moins un dérivé de la quinazolinone selon la revendication 1.

8. Utilisation des dérivés de la quinazolinone selon la revendication 1, pour la préparation de médicaments.

9. Utilisation selon la revendication 8, pour la préparation d'agents anti-inflammatoires, analgésiques et/ou sédatifs.